# EUROPEAN PATENT APPLICATION

(11) **EP 4 563 075 A1**
(43) Date of publication of application: **04.06.2025**
(21) Application number: 23212763.9
(22) Date of filing: 28.11.2023
(51) Int. Cl.: A61B 5/00, A61B 5/021, A61B 5/117, A61B 5/1455, A61B 5/1172, G06F 21/32, G06F 21/62, G16H 10/60

(54) **INTEGRATED HEALTH MONITORING HUB WITH SYNCHRONIZED DATA COLLECTION AND USER AUTHENTICATION**

(71) Applicant: AIKON Technologies B.V., 5656 AE Eindhoven (NL)
(72) Inventor: KANAGASABAPATHI, Thirukumaran Thangaraj, 5656 AE Eindhoven (NL); KJELLANDER, Birgitta Katarina Charlotte, 5656 AE Eindhoven (NL); SCHULING, Franklin Harold, 5656 AE Eindhoven (NL)
(74) Representative: V.O.

(57) **Abstract**

The invention relates to a hub configured to wirelessly connect to a plurality of wearable devices of one or more users, each wearable device being attachable to a part of a body for collecting health-related data, wherein the hub comprises a photoplethysmogram, PPG, sensor, and wherein an optical window of the PPG sensor is arranged on the hub, wherein a user-identification sensor is arranged at the PPG sensor, the user-identification sensor being configured to authenticate a user identity.

## Description

### FIELD OF THE INVENTION

The invention relates to a hub configured to wirelessly connect to a plurality of wearable devices of one or more users, each wearable device being attachable to a part of a body for collecting health-related data. Furthermore, the invention relates to a method of configuring a hub wirelessly connectable to a plurality of wearable devices of one or more users, each wearable device being attachable to a part of a body for collecting health-related data. Additionally, the invention relates to a modular system comprising a hub and modular wearable devices wirelessly connectable to the hub. Also, the invention relates to a method of performing user-authenticated health data collection and communication using a hub and a plurality of wearable devices of one or more users communicatively coupled to the hub.

### BACKGROUND TO THE INVENTION

Modern healthcare relies heavily on technology to monitor and manage patient health. Wearable devices have become increasingly popular for tracking various health parameters, such as heart rate, activity levels, and/or sleep patterns. However, existing systems present several challenges that can compromise the effectiveness of health monitoring and the convenience for the user.

One of the primary issues with current wearable technology is the lack of a centralized system for data collection and management. Users often need to interact with multiple devices, each with its own set of controls and data outputs, which can lead to confusion and data fragmentation. The dispersed nature of this data makes it difficult to achieve a comprehensive view of the user's health, potentially impacting the accuracy of health assessments.

Furthermore, the existing wearable devices often operate independently, resulting in complex synchronization algorithms for achieving synchronized data collection. This asynchrony can result in significant time lags between data sets from different devices, undermining the temporal accuracy of health information. Inconsistent data due to lack of synchronization may lead to misinterpretation of health status and, consequently, to suboptimal health management.

Another significant problem is the identification of the user, especially in scenarios where multiple individuals use the same device or system, this can lead to erroneous association of health data with user profiles, compromising data integrity and user privacy.

The current health monitoring systems typically do not offer adaptability in measurement parameters or user-specific health management plans. They are often rigid in their operation, unable to adapt to the changing health needs or preferences of individual users. The solutions in the art can be less effective for personalized health management and may impact user adherence to health monitoring regimes, medication regimes, consistent, accurate monitoring of vital signs, etc. These are hurdles that are particularly acute in the management of chronic diseases and conditions.

At present, health monitoring devices encompass a myriad of separate components. The efficacy of such devices hinges on their ability to communicate with central computing devices, like smartphones or tablets, to facilitate the transfer and synchronization of health data. Ultimately, this data must be uploaded to cloud-based systems for further processing and evaluation.

However, this multistep process presents several layers of complexity for users, especially among elderly patients, who often bear the brunt of chronic health issues. The necessity of operating multiple devices, each with its own interface and synchronization requirements, adds to the cognitive and physical burden on these patients. This complexity is compounded by the requirement to interact with modern mobile technology, which may be less intuitive for older users.

Moreover, the current devices require manual initiation and operation for each use, a process that can be cumbersome and time-consuming. This requirement for active patient engagement in the operational aspects of these devices, including the need to manually sync and upload data, has been identified as a significant barrier to adherence. The result is a discordance between the potential benefits of the health monitoring technology and its practical implementation among the patient population most in need of such monitoring.

The resultant low adherence to telemonitoring protocols not only undermines the effectiveness of chronic disease management but also diminishes the capacity of healthcare providers to deliver timely interventions based on accurate and up-to-date patient health data. In light of these issues, there exists a need for a system that simplifies the process, fostering greater patient engagement, and streamlining the data collection and transmission process to enhance adherence and ultimately improve patient outcomes.

In light of the above challenges, there is a clear need for an improved approach to health data collection and management.

### SUMMARY OF THE INVENTION

It is an object of the invention to provide for a method and a system that obviates at least one of the above mentioned drawbacks.

Additionally or alternatively, it is an object of the invention to provide for a hub for wireless connection to multiple wearable devices with improved operation.

Additionally or alternatively, it is an object of the invention to provide a method and system that improves the accuracy and/or reliability of health data collection using a centralized hub.

Additionally or alternatively, it is an object of the invention to streamline the user experience in health monitoring and better adapt to individual user needs.

Thereto, the invention provides for a hub configured to wirelessly connect to a plurality of wearable devices of one or more users, each wearable device being attachable to a part of a body for collecting health-related data, wherein the hub comprises a photoplethysmogram, PPG, sensor, and wherein an optical window of the PPG sensor is arranged on the hub, wherein a user-identification sensor is arranged at the PPG sensor, the user-identification sensor being configured to authenticate a user identity.

The hub wirelessly connects to multiple wearable devices of one or more users. This can be done simultaneously. Each wearable device may be attachable to a part of a body of a user for collecting health-related data. The photoplethysmogram (PPG) sensor is incorporated within the hub, enhancing the ability to collect vital physiological data. The optical window of the PPG sensor is arranged on the hub, enabling convenient and efficient data acquisition. Moreover, the integration of a user-identification sensor at the PPG sensor serves the dual purpose of user authentication and streamlined data collection, ensuring that the health-related data is securely associated with the correct user profile. The placement of the finger on the optical window is used for both identification and measurement, thereby simplifying the operation and improving the design of the hub.

The user-identification mechanism integrated into the health monitoring hub represents a multifaceted feature that transcends mere identity recognition. It acts as a proximity sensor, providing an indication of the presence of an identified user to the hub. In this way, the communication between the hub and the body-attached sensors of said identified user can be better guaranteed during a measurement process.

The proximity awareness can thus facilitate real-time monitoring by ensuring that the hub and sensors operate within optimal range for data fidelity, and it can mitigate the likelihood of errors that can arise from signal attenuation or interference often encountered in wireless communications. The strategic placement and operation of the user-identification sensor can thus serve to maintain a robust connection between the hub and the wearable sensors. As such, the data integrity of vital sign measurements is preserved, enabling dependable tracking of the user's health in real-time scenarios.

Furthermore, this user-identification feature is not just a static function; it actively interacts with the user to optimize the conditions for data acquisition. It can be used to guide users into positions or states that are most suitable for accurate sensor readings. For example, the hub may utilize a visual display or audio cues to encourage the user to sit down and relax their arm for a blood pressure reading, or to stand still when measuring weight or balance. This is especially pertinent for sensors that measure parameters prone to noise and inaccuracies caused by excessive motion or poor sensor contact, such as optical heart rate monitors, accelerometers, etc.

By directing the user to minimize movement, the hub can reduce the noise-to-signal ratio in the collected data, a common pitfall in wearable health monitoring. This not only can enhance the precision of each measurement but also contributes to the generation of high-quality longitudinal health data, which can be important for monitoring trends and detecting deviations indicative of health issues.

The hub's capability to condition the user's behavior and environment effectively transforms passive monitoring into an interactive and adaptive health management experience.

Furthermore, the integration of a user-identification sensor at the PPG sensor capitalizes on the action of a user placing a finger on the optical window of the PPG sensor to serve two pivotal roles: firstly, it authenticates the user's identity, and secondly, it initiates the collection of health-related data. This duality of function is not only efficient but also ensures that the data collected is securely associated with the correct user profile, thereby maintaining the integrity of personalized health data. On one hand, the user experience can be enhanced by reducing the complexity of the operation - a single action of placing a finger on the sensor is all that is needed to unlock the device and begin data collection. This effectively streamlines the process for the user, eliminating the need for separate steps of user authentication and data recording, which might otherwise be cumbersome or time-consuming. On the other hand, this integrated approach significantly improves the design of the hub. By combining two functions into one contact point on the device, it minimizes the hardware requirements, saving space and resources and potentially reducing the cost and complexity of the hub's manufacturing.

The proximity of the user-identification sensor to the PPG sensor also enhances the speed and accuracy of the data synchronization process, as the authentication and the initiation of data collection occur almost simultaneously. This results in a more efficient system that can promptly begin to collect health data immediately following authentication, minimizing any delays that could affect the data's accuracy or relevance.

Furthermore, the user-identification sensor's co-location with the PPG sensor mitigates the risk of data misattribution or unauthorized access. By confirming the user's identity at the point of measurement, the system ensures that health data is accurately logged and attributed in real-time, enhancing the privacy and security of sensitive health information. This secure linkage between the user's biometric identity and their health data underscores the system's robustness against potential data breaches or user impersonation.

Optionally, the user-identification sensor is integrated or embedded at the PPG sensor.

This consolidation of functionality into a single, unified component effectively reduces the spatial footprint of the hub's assembly. This space efficiency not only contributes to the miniaturization of the hub but also simplifies its overall architecture.

When users interact with the hub, specifically when they place a finger on the PPG sensor for a reading, their identity is verified in the same motion required for health data collection. This eliminates additional steps for the user, thereby enhancing the user experience through a more intuitive and less intrusive process. It simplifies the user's interaction with the device to a single, effortless action, which is a significant consideration in designing user-friendly health monitoring devices.

Integrating or embedding the user-identification sensor within the PPG sensor at its optical window achieves a compact and efficient design that enhances the system's reliability and ease of use.

Alternatively, the user-identification sensor can be a separate component that is in close proximity to the PPG sensor, yet functionally connected to provide seamless user identification and data collection.

In some examples, the hub may include a user-identification sensor that employs biometric modalities or verification methods to authenticate user identity. A combination of biometric modalities or biometric verification methods may also be used.

Optionally, the user-identification sensor is a fingerprint sensor arranged within the optical window of the PPG sensor, wherein the fingerprint sensor is configured to authenticates the patient's identity when a user places a finger on the optical window, and wherein the hub is configured to, once the user is authenticated, initiate a measurement using at least the PPG sensor.

The fingerprint sensor within the optical window of the PPG sensor enables a secure authentication of the user's identity and immediate initiation of health measurement upon verification. By situating the fingerprint sensor within the PPG sensor's optical window, the invention maximizes functional integration, allowing a single contact point for both identity verification and biometric data collection.

By requiring fingerprint authentication to access the device's functionality, the system ensures that health measurements are inseparably linked to the verified user, thus maintaining the integrity and privacy of sensitive health information. This security measure is important in healthcare applications where unauthorized access to personal health data is a significant concern.

Moreover, this integration of sensors enhances the usability of the hub. Instead of separate actions for identification and measurement, which can complicate and lengthen the process, the user completes both by placing a finger on the optical window. This not only streamlines the operational procedure but also reduces the time taken from the user's perspective to engage with the device. By simplifying the process, the hub becomes more accessible, especially to users who may have limitations that make complex interactions with technology challenging.

Furthermore, the hub can be effectively used to serve a relatively large number of users, each having a personalized profile.

It will be appreciated that the integrated sensor may utilize capacitive, optical, ultrasonic, or thermal fingerprint sensing technologies, among others, to facilitate user authentication.

Additionally or alternatively, other biometric modalities and/or verification methods for authenticating user identity can also be used. Examples include voice recognition, retinal scanning, etc.

In some examples, the fingerprint sensor may be arranged adjacent to the optical window or other biometric sensors may be incorporated within the optical window that use different physiological or behavioral characteristics to authenticate the user.

Optionally, the hub comprises synchronization module for sending a trigger signal to the wearable devices to initiate and synchronize measurements when time-synchronized data is required, said synchronization module being activated based on the placement of a finger on the optical window of the PPG sensor.

The synchronization module may be used for coordinating the operation of at least a subset of the wearable devices. The synchronization module can send a trigger signal to the wearable devices to initiate and synchronize measurements when time-synchronized data is required. By activating the synchronization module based on the placement of a finger on the optical window of the PPG sensor, the hub streamlines the process of data synchronization, ensuring accuracy and temporal correlation of the collected data.

The inclusion of a synchronization module within the hub enables orchestrating the synchronized operation of multiple wearable devices. The trigger signal that is dispatched to the connected wearable devices, can prompt them to initiate and harmonize their measurement activities. This can ensure that data collected is temporally aligned/congruent, providing a coherent and comprehensive health profile of the user.

The synchronization module may be activated via the action of placing a finger on the PPG sensor's optical window. Additionally or alternatively, the trigger for synchronization can be activated by other means, such as a verbal command, a gesture, and/or automatically upon detection of the user's biometric data.

Optionally, the hub comprises an internal clock used for synchronizing measurements, wherein, upon execution of the measurement utilizing at least the PPG sensor, the hub's internal clock is configured to generate a timestamp, and wherein the hub is configured to establish wireless communication with other wearable devices associated with the identified user, and send requests to said wearable devices, specifying the timestamp as a reference, and wherein, in response to the requests, the wearable devices transmit their stored data corresponding to the specified timestamp, to the hub.

The hub can have an internal clock used for synchronizing measurements. The internal clock may generate precise timestamps that correlate with the execution of measurements using the PPG sensor. The hub establishes wireless communication with other wearable devices associated with the identified user and orchestrates data collection across devices using the timestamp as a reference. This systematic approach ensures temporal accuracy and coordination of health data from multiple sources, enabling the integration of this data with high precision.

The integration of an internal clock within the hub can be important for facilitating the synchronization of health data measurements across multiple wearable devices. The internal clock can be important to the hub's operation, serving as the standard reference for all timing and synchronization processes.

When the hub communicates with the associated wearable devices, it can transmit requests that include these timestamps, instructing the devices to provide data that aligns temporally with the measurements taken by the PPG sensor. By ensuring that all data points from the different sensors are timestamped in a synchronized manner, the hub allows for an integrated health data profile where the temporal relationships between different physiological signals are maintained.

The temporal alignment is especially significant when monitoring for events that require the analysis of simultaneous data streams, such as the assessment of cardiovascular health where the synchronization of heart rate (ECG) and blood oxygenation (PPG) data is important.

Optionally, the hub collects the transmitted data from the wearable devices and the measured data from the PPG sensor, and stores both sets of data with the associated timestamp, wherein the hub further integrates the collected data, ensuring synchronization and correlation between the measurements from the PPG sensor and the transmitted data from the wearable devices based on the timestamp.

The hub can collect transmitted data from wearable devices and measured data from the PPG sensor and store the sets of data with the associated timestamp. A reliable synchronization and correlation of health data from disparate sources can be obtained, enhancing the integrity and utility of the collected data. By integrating the data with timestamp correlation, the hub ensures that health assessments are based on synchronized data points, providing a robust foundation for accurate health analysis.

The hub can effectively synchronize data from multiple, heterogeneous sources, thereby constructing a cohesive and comprehensive dataset that reflects the synchronous nature of physiological processes. The temporal alignment is important when analyzing physiological events that are inherently dependent on the timing of various bodily functions, such as correlating heart electrical activity with peripheral blood flow. Secondly, the storage of both sets of data with associated timestamps ensures that the health data can be analyzed with a clear understanding of the sequence and simultaneity of physiological events.

Optionally, at least one of the wearable devices is an ECG sensor, and wherein data collected using the ECG sensor and data collected using the PPG sensor is timestamped and correlated to measure data indicative of the pulse arrival time (PAT).

Timestamping and correlating ECG and PPG data enable precise determination of PAT, a valuable metric for assessing cardiovascular health. This accurate measurement is made possible by the hub's synchronization capabilities, which align the data collection events from both sensor types.

The hub not only communicates with a variety of wearable devices but also specifically includes the capability to interface with an ECG sensor. The ECG sensor, which detects the electrical activity of the heart, is a critical component in the assessment of cardiovascular health. When used in conjunction with the PPG sensor integrated in the hub, and which optically measures blood volume changes, the hub can accurately determine the PAT.

The synchronization is important because PAT is measured as the time difference between the waveforms produced by these two sensors. For the PAT to be accurately determined, the time stamps of the ECG waveform and the PPG waveform must be precisely aligned. This advantage is realized through the hub's internal clock, which serves as the central reference point for all timestamping processes, ensuring that the data from both sensors are perfectly synchronized.

The ability to correlate the exact moments of cardiac electrical activity with the resultant blood volume changes in peripheral tissues enhances the diagnostic value of the collected data. Secondly, by enabling the precise determination of PAT, the hub can assist in detecting changes in arterial stiffness, a factor that can indicate various cardiovascular conditions.

Advantageously, the PAT measurement, which is an indicator of the time it takes for the blood pulse to travel from the heart to peripheral sites, can be calculated with high accuracy. This measurement is valuable in both acute and long-term health monitoring scenarios and can lead to early detection of cardiovascular abnormalities. The accurate determination of PAT can also be instrumental in evaluating the effectiveness of medical interventions, monitoring disease progression, or even the impact of lifestyle changes on cardiovascular health.

In some examples, the hub may be configured to employ different types of sensors, such as a blood pressure sensor or an accelerometer, to gather additional or alternative data to be correlated with the PPG sensor data.

Optionally, the ECG sensor is configured to detect electrical activity of the heart and produce an ECG waveform, wherein the PPG sensor is configured to optically measure blood volume changes in the microvascular bed of tissue and produce a PPG waveform, and wherein the internal clock of the hub is configured to provide synchronized timestamping for both the ECG waveform and the PPG waveform, and wherein a processing unit of the hub is configured to calculate the PAT based on the synchronized timestamped data from both the ECG and PPG waveforms.

The processing unit can be configured to calculate PAT by analyzing the timing relationship between the ECG and PPG waveforms. Both the ECG sensor, which records the heart's electrical activity, and the PPG sensor, which monitors blood volume changes, produce waveforms that are critical to determining PAT. The precise calculation of PAT requires the exact moment-to-moment correlation between these two waveforms, which is possible only if they are perfectly synchronized. This synchronization is achieved through the hub's internal clock, which timestamps both the ECG and PPG data. The processing unit then uses these synchronized timestamps to determine the exact time delay between the heart's electrical activity (detected by the ECG sensor) and the change in blood volume in the periphery (detected by the PPG sensor).

In some examples, the hub may, additionally or alternatively, employ different techniques for detecting heart activity or blood volume changes, such as bioimpedance or acoustic methods, and calculate corresponding time-based health metrics.

Optionally, the processing unit of the hub is configured to derive PAT as the time difference between a point on the ECG waveform, and a point on the PPG waveform.

By accurately identifying the relevant points on both ECG and PPG waveforms, the hub can measure the time taken for the pulse wave to travel from the heart to the peripheral site, which is the PAT. The hub's processing unit can calculate the PAT by determining the time difference between the aforementioned points on the ECG and PPG waveforms. This requires high-resolution timestamping and advanced signal processing algorithms to ensure accuracy.

The processing unit can be configured to deduce the PAT as the temporal differential between a selected point on the ECG waveform and a corresponding point on the PPG waveform. In some examples, additionally or alternatively, the processing unit may be configured to employ algorithms or artificial intelligence (e.g. machine learning models) to identify the points more accurately or to deduce additional health-related metrics from the waveform data.

Optionally, the PPG sensor is configured to measure SpO2 in reflection mode by analyzing light reflected from a fingertip placed over the optical window during use.

This provides a non-invasive and convenient assessment of the user's oxygen saturation levels. The PPG sensor's configuration for reflection mode measurement exploits the natural action of placing a finger over the optical window, simplifying the process for users and enabling immediate and accurate SpO2 readings. SpO2 is a measure of how much oxygen the blood is carrying as a percentage of the maximum it can carry, which is important for monitoring respiratory and cardiovascular health.

The PPG sensor only needs light to be reflected back from the blood-perfused tissue, in this example a fingertip. By utilizing the natural action of placing a finger on the sensor, the device simplifies the user experience. There's no need for additional equipment or complex procedures, making it more likely that individuals will regularly monitor their oxygen levels.

The measurement of SpO2 can be done quickly, providing immediate feedback to the user. This immediacy is important for individuals who need to monitor their oxygen levels frequently, such as those with chronic respiratory conditions.

Since the sensor also serves other functions, such as user identification and possibly heart rate measurement, its use for SpO2 measurement adds to the device's multifunctional capabilities without adding bulk or complexity.

Optionally, the hub further comprises a control unit configured to modify measurement schemes for the identified user, wherein said modification includes at least one of adjusting the frequency of measurements, changing the set of parameters to be measured, changing one or more measurement types, and/or altering the duration of measurement periods.

The control unit may be configured to modify measurement schemes for the identified user. A personalized and adaptive management of health monitoring can be obtained. By adapting the measurement schemes to the individual user, the hub can cater to specific health needs and concerns. For example, a person with a known heart condition may require more frequent ECG readings, whereas an athlete may focus on oxygen saturation and heart rate during various stages of their training.

The control unit can determine how often measurements should be taken. This can range from continuous monitoring to periodic checks throughout the day.

Depending on the user's health profile, the hub can select which types of data are most relevant. For instance, it may prioritize cardiovascular data for a heart patient or activity levels for a user focusing on physical fitness.

The duration of each measurement session can also be tailored. Longer durations may be necessary for capturing more detailed data over a period, while shorter ones can suffice for quick checks.

The hub can establish a monitoring routine that balances the need for detailed health data with the user's comfort and daily life disruptions. It can avoid unnecessary measurements while ensuring critical data is collected when needed.

The hub can better handle chronic condition management, where monitoring needs can change over time based on the user's condition and response to treatment.

By providing a tailored health monitoring experience, users are more to engage with the device and comply with the recommended health monitoring routines, thereby improving health outcomes.

In some examples, additionally or alternatively, the modifications can be determined based on machine learning algorithms that adapt to the user's historical data or external data sources, such as environmental sensors and/or a connected health record system.

Optionally, the control unit is configured to notify the identified user of drug regimen changes via a communication interface.

The hub may be configured to notify the identified user of drug regimen changes based on collected and/or analyzed health data.

The health monitoring hub can provide for a personalized approach to health management by being programmed to alert the identified user of modifications required in their medication schedule in response to the analyzed health data. The hub, through its integrated user-identification sensor, ensures that the health data collected is accurately attributed to the correct user profile. This precise attribution is essential because medication regimens are highly individualized, and adjustments can be made based on the specific health readings and trends of the individual.

Once health data is gathered by the hub, whether it's heart rate, blood pressure, activity levels, or other biometrics, it is processed to detect any deviations or trends that might warrant a change in medication. This could include for example detecting changes in heart rhythm that may affect how a user should use their antiarrhythmic medications.

When such changes are identified, the hub may be configured to use its communication capabilities to inform the user of the necessary adjustments to their drug regimen.

In some examples, the communication interface also allows for a two-way interaction where users can acknowledge receipt of notifications or input information related to their medication intake, providing a feedback loop for better management.

Integrating drug regimen notifications means that the hub is not just a passive collector of health data but also an active participant in the user's health care.

It will be appreciated that the communication interface can utilize various communication protocols, such as in-app notifications, SMS, email, audio, video, etc. In some examples, additionally or alternatively, the notification system can be expanded to include voice-activated alerts, haptic feedback, or visual signals on a connected device.

In some examples, the communication interface includes at least one of a visual display, an audio output, or a feedback mechanism on the hub to provide the user with immediate notification of drug regimen changes. In some examples, the visual display is a touch screen for user interactions. The display may for example show medication information, vital signs and other relevant information. For instance, the hub may in this way provide messages from the health care providers.

In some examples, the hub is configured to send data to a remote location, for example the cloud. This can be through various communication means, such as cellular networks, Wi-Fi, wired network, etc.

In some examples, the control unit is further configured to transmit information regarding the modified measurement schemes or drug regimen changes to an external device, such as a smartphone or a computer, owned or operated by the user or a healthcare provider.

In some examples, the modification of the measurement schemes or drug regimen changes are performed automatically by the hub using a machine learning algorithm that processes the user's authenticated health-related data over time to optimize the user's health management.

In some examples, the external device comprises an application configured to allow the user or the healthcare provider to manually override or adjust the suggested measurement schemes or drug regimen changes proposed by the hub's control unit.

In some examples, the hub is configured to notify the user of drug regimen changes via the hub based on the modified measurement schemes and the collected health-related data, wherein the hub utilizes a communication interface for such notifications.

Optionally, the hub is usable for recharging wearable devices.

By serving as a charging station, the hub facilitates the management of wearable devices, reducing the likelihood of downtime due to power depletion.

By combining data processing with the ability to recharge wearable devices, the hub eliminates the need for separate charging equipment. This consolidation reduces clutter and simplifies the user's routine since they can manage their health monitoring and device maintenance in one place.

The convenience of having a single point to manage and charge wearable devices may increase user adherence to prescribed health monitoring regimes. If it's easy to charge and manage the devices, users are more to use them regularly and consistently.

Optionally, the hub is designed to serve multiple users.

Optionally, the control unit is used as a communication interface that allows two-way interaction.

In some examples, the control unit may be configured to receive and process incoming data and to send data (e.g. feedback, responses, or alerts) back. This bi-directional communication capability is pivotal for applications requiring real-time interaction.

Optionally, the hub is used for sending data to the cloud through cellular networks, Wi-Fi or internet connectivity.

The hub may be configured to interface with various types of network connectivity. This includes not only traditional cellular networks, which offer widespread coverage and reliability, but also Wi-Fi networks, known for their highspeed data transfer capabilities within localized areas. Additionally or alternatively, the hub can connect to broader internet services, allowing for a seamless and efficient transmission of data to cloud-based platforms. This flexibility in connectivity options ensures that the hub can adapt to different environments and user requirements, whether it's in a residential setting, a commercial environment, or even in mobile or remote locations. In some examples, the hub is configured to choose the most suitable connection type based on availability and efficiency makes it an ideal solution for a wide range of applications, ensuring consistent and reliable data transmission to cloud services.

According to an aspect, the invention provides for a method of configuring a hub wirelessly connectable to a plurality of wearable devices of one or more users, each wearable device being attachable to a part of a body for collecting health-related data, wherein the hub is provided with a photoplethysmogram, PPG, sensor, and wherein an optical window of the PPG sensor is provided on the hub, and wherein a user-identification sensor is provided at the PPG sensor, the user-identification sensor being used to authenticate a user identity.

The integration of a user-identification sensor with the PPG sensor enhances the hub's design significantly. These two sensors are cohesively combined within the central hub, offering a streamlined user experience. Positioned to intercept a finger for biometric readings, the optical window of the PPG sensor is advantageously used for incorporating a user-identification sensor. This placement not only can facilitate immediate user recognition but also can circumvent at least some complexities associated with time synchronization among various sensors. The central hub's internal clock may serve as the universal timing reference for performing synchronization.

According to an aspect, the invention provides for a modular system comprising the hub according to the disclosure and modular wearable devices wirelessly connectable to the hub.

According to an aspect, the invention provides for a method of performing user-authenticated health data collection and communication using a hub and a plurality of wearable devices of one or more users communicatively coupled to the hub, each wearable device being attachable to a part of a body for collecting health-related data, the method comprising the steps of: establishing a wireless connection between the hub and at least a subset of the plurality of wearable devices of the one or more users; providing a photoplethysmogram (PPG) sensor integrated within the hub, wherein an optical window of the PPG sensor is arranged on the hub to collect optical data; activating a user-identification sensor arranged at the PPG sensor; authenticating a user identity based on data received from the user-identification sensor; and collecting health-related data from the plurality of wearable devices upon successful authentication of the user identity.

The central hub serves as the core of the health data management system, integrating data from various wearable devices. By linking the initiation of data collection to user authentication, the system ensures that data is not only collected securely but is also correctly attributed to the user's profile, minimizing errors and enhancing personalized health monitoring.

According to an aspect, the invention provides for a patient monitoring hub, comprising: an embedded optical window; a photoplethysmogram (PPG) sensor integrated within said optical window configured to measure blood oxygen saturation (SPO2) levels of a patient; a fingerprint sensor integrated within said optical window configured to authenticate a patient's identity; and a communication module configured to retrieve and synchronize data from other wearable medical devices based on the timestamp of the SPO2 measurement.

Optionally, the hub includes a display configured to visualize synchronized patient data, including said SPO2 levels.

Optionally, the hub generates a timestamp concurrent with the initiation of an SPO2 measurement, said timestamp used to correlate and synchronize data from said wearable medical devices.

Optionally, the hub includes color-coded LED indicators, signifying the initiation and completion of SPO2 measurements.

Optionally, the hub is configured to provide instructions, reminders, and notifications to the patient based on the collected and synchronized data.

According to an aspect, the invention provides for a method for synchronized patient monitoring, comprising: authenticating a patient's identity using a fingerprint sensor integrated within an optical window of a patient monitoring hub; initiating an SPO2 measurement using a PPG sensor integrated within said optical window upon successful authentication; generating a timestamp concurrent with said SPO2 measurement; and synchronizing and retrieving data from wearable medical devices based on said timestamp.

Optionally, the method further includes visually presenting the synchronized data, including the SPO2 measurement, on a display of the patient monitoring hub.

Optionally, the wearable medical devices include devices configured to measure user characteristics including but not limited to ECG, heart rate, body movement/acceleration, body posture or body temperature.

It will be appreciated that any of the aspects, features and options described in view of the hub apply equally to the methods, system and the described devices. It will also be clear that any one or more of the above aspects, features and options can be combined.

### BRIEF DESCRIPTION OF THE DRAWING

The invention will further be elucidated on the basis of exemplary embodiments which are represented in a drawing. The exemplary embodiments are given by way of non-limitative illustration. It is noted that the figures are only schematic representations of embodiments of the invention that are given by way of non-limiting example.

In the drawing:
Fig. 1 shows a schematic diagram of a cross-sectional side view of an embodiment of a hub;
Fig. 2 shows a schematic diagram of a cross-sectional side view of an embodiment of a hub;
Fig. 3 shows a schematic diagram of a top view of an embodiment of a hub;
Fig. 4 shows a schematic diagram of a perspective view of an embodiment of a hub; and
Fig. 5 shows a schematic diagram of a method.

### DETAILED DESCRIPTION

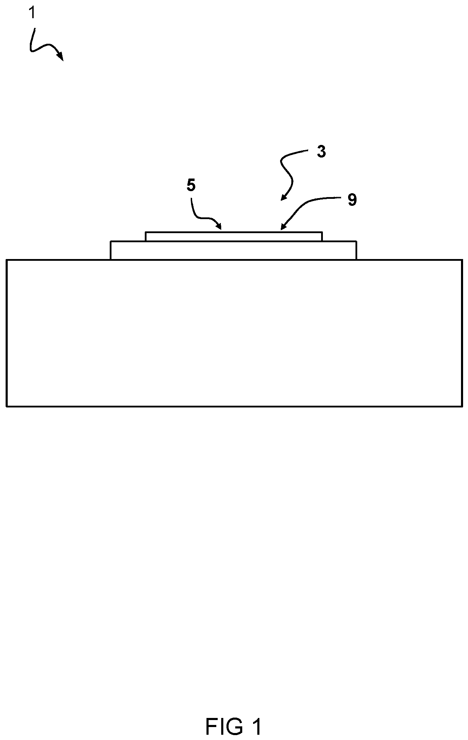

Fig. 1 shows a schematic diagram of a cross-sectional side view of an embodiment of a hub 1. The hub 1 is configured to wirelessly connect to a plurality of wearable devices of one or more users, wherein each wearable device is attachable to a part of a body for collecting health-related data. The hub 1 comprises a photoplethysmogram, PPG, sensor 3, and wherein an optical window 5 of the PPG sensor 3 is arranged on the hub 1. Furthermore, a user-identification sensor 7 is arranged at the PPG sensor 3. The user-identification sensor 7 is configured to authenticate a user identity. In this example, the user-identification sensor 7 is integrated or embedded at the PPG sensor 3.

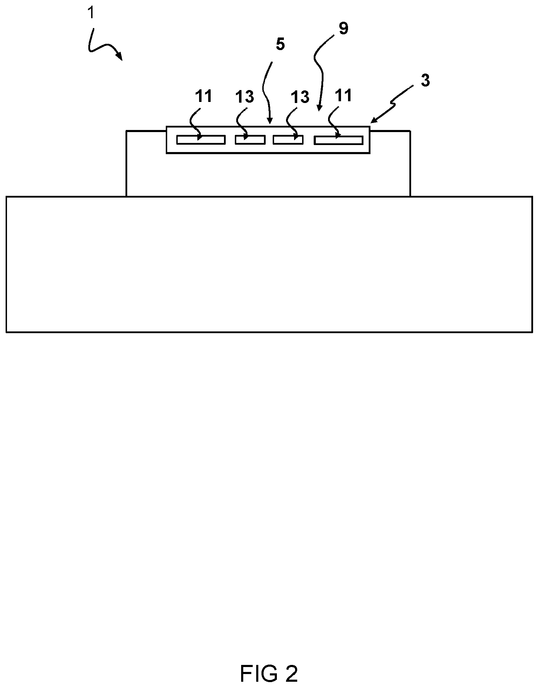

Fig. 2 shows a schematic diagram of a cross-sectional side view of an embodiment of a hub 1. The hub has a PPG sensor 3 with an optical window 9 on its top side. In this example, the hub has an electronic unit that has readout electronic arrangements. The hub may comprise one or more power sources. For example, the hub may include a rechargeable battery unit. However, a power source via a wired connection is also possible. In some examples, the hub may include one or more other sensors, such as accelerometers, temperature sensors, etc.

In this example, the PPG sensor 3 has a transparent optical window 5. For example, the optical window 5 may be made of glass or any other suitable material.

In this example, the PPG sensor 3 has two light emitting units 11, such as light emitting diodes (LEDs), and two optical sensors 13, such as photodiodes. Various other arrangements and configurations are possible.

To make a measurement, patients are required to place their fingers to cover the optical window on the device. Once the finger is placed on the device, depending on the skin type and/or other predetermined light-skin interaction models, a particular wavelength of LED light is enabled in the PPG module 3. The reflected light from the skin is measured by one or more photodiodes located on the top surface of the hub.

In some examples, the PPG module includes one or more LEDs of different wavelengths (Blue, Green, Red and Infra-red) and one or more photodiodes to measure the reflected light from the skin.

Whenever the patient is required to make a SPO2 measurement, the patient will place a finger on top of the optical window 5. On contact, the LED gets activated, the reflected light from the finger will be measured by photodiodes in the center of the PPG module. Depending on the skin type, a particular wavelength of LED light will be used for measurement. Furthermore, when the finger is placed on the optical window 5, a fingerprint sensor 9 authenticates the patient's identity. The fingerprint sensor 9 is arranged at the optical window 5.

One or more LEDs can be used in sequence and the best signal output with low signal-to-noise ratio depending on the skin type can be used for SPO2 calculation. A capacitive touch sensor can be included in the PPG module to activate the LED lights on contact. However, other mechanisms may also be used.

In some examples, the measurement protocol is only activated when the user is authenticated by the fingerprint sensor 9, to avoid accidental light activation.

In some examples, SPO2 is measured in reflection mode by measuring the light reflected from the finger-tip. The invention avoids the need to have a wired finger clip sensor to be connected to the fingers each time. SPO2 can be measured on demand by placing the fingers on top of the hub.

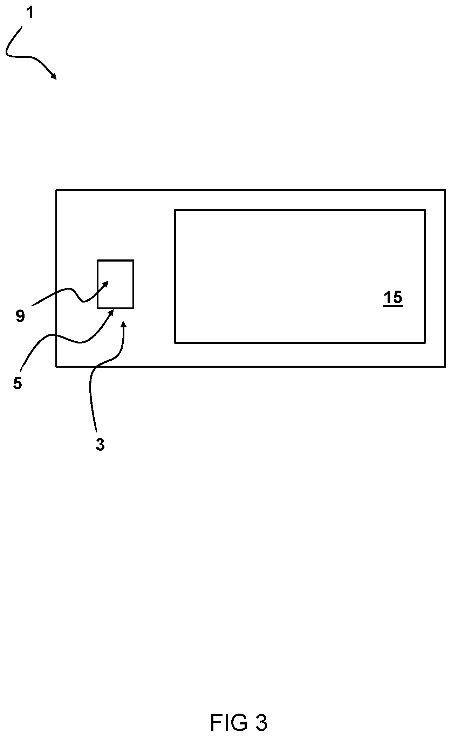

Fig. 3 shows a schematic diagram of a top view of an embodiment of a hub 1. In this example, the hub has a window 15 used as a communication interface. In some examples, this window 15 has a touch screen, enabling a two-way communication. The hub may be configured to open and display the user profile once the user has been authenticated. The user may manually lock the device. In some advantageous examples, the user is automatically logged out based on a distance of one or more sensors attached to its body and the hub.

The hub 1 can effectively improve the patient monitoring process by integrating SPO2 measurements directly within the hub. In some advantageous examples, the hub may use this SPO2 measurement as a trigger point to synchronize and gather data from various wearable sensors, ensuring a comprehensive and correlated view (cf. temporal alignment) of the patient's health at any moment.

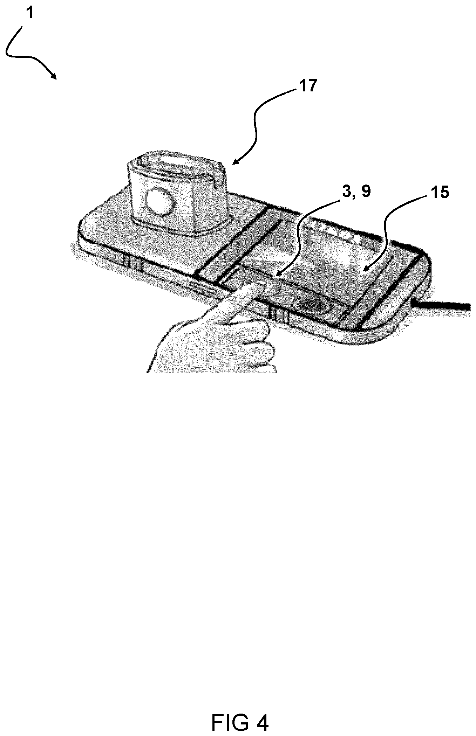

Fig. 4 shows a schematic diagram of a perspective view of an embodiment of a hub 1. The PPG sensor 3 has an optical window 5 at which the fingerprint sensor 9 is arranged.

In this example, the hub 1 has a recharge port 17 that can be used for recharging wearable sensors. Advantageously, the recharge port 17 has a complementary shape in order to facilitate placement of the one or more wearable sensors.

In the shown example, the hub 1 has a screen 15 integrated in its body. The hub may be configured to wirelessly or wiredly communicate with one or more other devices and/or networks, such as for instance the internet, the cloud, smartphones, computers, etc.

The hub 1 may thus be used for spot measurements of SPO2, patient interactions, patient notifications, and even charging. Also, the hub is configured to act as a central unit for collecting data wirelessly from multiple wearable devices of one or more users.

The centralized hub 1 may be used transmit the data to the cloud repository for post-processing. In some examples, the centralized hub is equipped with additional sensing units such as the temperature, capacitive sensors, bioimpedance, PPG sensors and fingerprint sensors to create interactive spot measurements for patients in remote telemonitoring. The hub 1 can be configured to prompt patients at set time interval to do measurements that are not part of the wearable device. In addition, the hub 1 can also send notifications to the patients at a pre-determined time for medication.

The interactive hub 1 can be used for spot measurements of patients vital signs that are not easy to measure with a wearable device.

In some examples, the centralized hub 1 includes data storage options to download data while charging, processor for post-processing / data analysis, cellular connectivity and an array of sensors such as temperature, bio-impedance, PPG, and finger print sensors. The hub may be equipped with a basic display for notifications and option to send timestamp of measurements taken. Reminders for medication can be provided, improving adherence to medication.

The interaction requirements for patients to do particular spot measurements that are not difficult to perform using a wearable device can be simplified. The hub 1 may act as a central node for receiving data wirelessly from the wearable device whilst also enabling important spot measurements. An intuitive single step spot measurement for blood oxygenation will require the patient to place their index finger on the optical window.

The finger print sensor embedded in the optical window will ensure patient's identity while measuring SPO2 .

In some examples, LEDs on the hub 1 are used to indicate when the measurement needs to be made and when measurements are complete. In some examples, based on the measurement requirements, the hub can be programmed remotely from the clinical dashboard to send notifications and pre-determined time interval.

Advantageously, the SPO2 sensor is integrated in the central hub 1, thereby eliminating the need for a standalone sensor. The data from the central hub 1 can be easily combined and timestamped with other wearable sensor data. For instance, every time when the patient makes a SPO2 measurement in the hub, corresponding ECG and other vital sign data can be timestamped and downloaded to the hub, thereby providing a comprehensive real-time view of the patient data at any given instance.

An advantageous data integration of SPO2 and other sensors in the centralized hub is obtained. The hub can download or receive data from other selected wearable devices when the user is identified. For example, instances of SPO2 measurement can be used as trigger points for downloading ECG data from wearable devices.

In some advantageous examples, the SPO2 measurement instances can be used as a timestamp for data correlation.

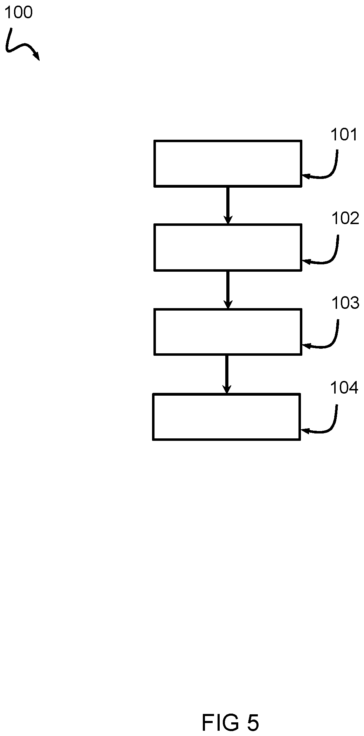

Fig. 5 shows a schematic diagram of a method 100 of performing user-authenticated health data collection and communication using a hub and a plurality of wearable devices of one or more users communicatively coupled to the hub, each wearable device being attachable to a part of a body for collecting health-related data. A photoplethysmogram (PPG) sensor integrated within the hub is provided, wherein an optical window of the PPG sensor is arranged on the hub to collect optical data. In a first step 101, a wireless connection between the hub and at least a subset of the plurality of wearable devices of the one or more users is established. In a second step 102, a user-identification sensor arranged at the PPG sensor. In a third step 103, a user identity is authenticated based on data received from the user-identification sensor. In a fourth step 104, health-related data from the plurality of wearable devices is collected upon successful authentication of the user identity.

In some examples, the central hub is designed with an embedded optical window. Within this optical window, PPG sensors are incorporated. These sensors typically use LEDs to transmit light (commonly red and infrared) through the finger. Photodiodes, also embedded, detect the light that passes through the finger or gets reflected back. The variation in light absorption across blood pulses provides the SPO2 readings. When a patient places their finger on the optical window, the fingerprint sensor authenticates the patient's identity. Once authenticated, the hub activates the SPO2 sensor, initiating the measurement.

In some examples, data synchronization is performed. As the SPO2 reading is taken, the hub's internal clock creates timestamps. Concurrently, the hub can communicate wirelessly with other wearable devices the patient might have (e.g., an ECG sensor). It requests data from these devices, specifying the time of the SPO2 measurement as a reference. The wearable devices may be configured to respond, transmitting their stored data, like ECG readings, from the specified timestamped period. The central hub collects this data and integrates it with the SPO2 reading. It stores both the SPO2 and the received data (like ECG) with the same timestamp, ensuring correlation between them. In this way, the measurements can be synchronized in an advantageous way, resulting in more accurate measurement results (for example for deriving PAT).

In some examples, the hub's onboard display can present the synchronized data in real-time, allowing for immediate insights. This could be numerical data, waveforms, or any relevant visuals. For more comprehensive views, the hub might also connect to a more extensive system, such as a hospital's patient monitoring dashboard. Data from the hub can be transmitted to this dashboard for detailed visualization and analysis.

In some examples, alert mechanisms can notify healthcare providers if any reading goes beyond a set threshold, ensuring quick response in emergencies.

It will be appreciated that the method may include computer implemented steps. All above mentioned steps can be computer implemented steps. Embodiments may comprise computer apparatus, wherein processes performed in computer apparatus. The invention also extends to computer programs, particularly computer programs on or in a carrier, adapted for putting the invention into practice. The program may be in the form of source or object code or in any other form suitable for use in the implementation of the processes according to the invention. The carrier may be any entity or device capable of carrying the program. For example, the carrier may comprise a storage medium, such as a ROM, for example a semiconductor ROM or hard disk. Further, the carrier may be a transmissible carrier such as an electrical or optical signal which may be conveyed via electrical or optical cable or by radio or other means, e.g. via the internet or cloud.

Some embodiments may be implemented, for example, using a machine or tangible computer-readable medium or article which may store an instruction or a set of instructions that, if executed by a machine, may cause the machine to perform a method and/or operations in accordance with the embodiments.

Various embodiments may be implemented using hardware elements, software elements, or a combination of both. Examples of hardware elements may include processors, microprocessors, circuits, application specific integrated circuits (ASIC), programmable logic devices (PLD), digital signal processors (DSP), field programmable gate array (FPGA), logic gates, registers, semiconductor device, microchips, chip sets, et cetera. Examples of software may include software components, programs, applications, computer programs, application programs, system programs, machine programs, operating system software, mobile apps, middleware, firmware, software modules, routines, subroutines, functions, computer implemented methods, procedures, software interfaces, application program interfaces (API), methods, instruction sets, computing code, computer code, et cetera.

Herein, the invention is described with reference to specific examples of embodiments of the invention. It will, however, be evident that various modifications, variations, alternatives and changes may be made therein, without departing from the essence of the invention. For the purpose of clarity and a concise description features are described herein as part of the same or separate embodiments, however, alternative embodiments having combinations of all or some of the features described in these separate embodiments are also envisaged and understood to fall within the framework of the invention as outlined by the claims. The specifications, figures and examples are, accordingly, to be regarded in an illustrative sense rather than in a restrictive sense. The invention is intended to embrace all alternatives, modifications and variations which fall within the scope of the appended claims. Further, many of the elements that are described are functional entities that may be implemented as discrete or distributed components or in conjunction with other components, in any suitable combination and location.

In the claims, any reference signs placed between parentheses shall not be construed as limiting the claim. The word 'comprising' does not exclude the presence of other features or steps than those listed in a claim. Furthermore, the words 'a' and 'an' shall not be construed as limited to 'only one', but instead are used to mean `at least one', and do not exclude a plurality. The term "and/or" includes any and all combinations of one or more of the associated listed items. The mere fact that certain measures are recited in mutually different claims does not indicate that a combination of these measures cannot be used to an advantage.

## Claims

1. A hub configured to wirelessly connect to a plurality of wearable devices of one or more users, each wearable device being attachable to a part of a body for collecting health-related data, wherein the hub comprises a photoplethysmogram, PPG, sensor, and wherein an optical window of the PPG sensor is arranged on the hub, wherein a user-identification sensor is arranged at the PPG sensor, the user-identification sensor being configured to authenticate a user identity.

2. The hub according to claim 1, wherein the user-identification sensor is integrated or embedded at the PPG sensor.

3. The hub according to claim 1 or 2, wherein the user-identification sensor is a fingerprint sensor arranged within the optical window of the PPG sensor, wherein the fingerprint sensor is configured to authenticates the patient's identity when a user places a finger on the optical window, and wherein the hub is configured to, once the user is authenticated, initiate a measurement using at least the PPG sensor.

4. The hub according to any one of the preceding claims, wherein the hub comprises synchronization module for sending a trigger signal to the wearable devices to initiate and synchronize measurements when time-synchronized data is required, said synchronization module being activated based on the placement of a finger on the optical window of the PPG sensor.

5. The hub according to any one of the preceding claims, wherein the hub comprises an internal clock used for synchronizing measurements, wherein, upon execution of the measurement utilizing at least the PPG sensor, the hub's internal clock is configured to generate a timestamp, and wherein the hub is configured to establish wireless communication with other wearable devices associated with the identified user, and send requests to said wearable devices, specifying the timestamp as a reference, and wherein, in response to the requests, the wearable devices transmit their stored data corresponding to the specified timestamp, to the hub.

6. The hub according to claim 5, wherein the hub collects the transmitted data from the wearable devices and the measured data from the PPG sensor, and stores both sets of data with the associated timestamp, wherein the hub further integrates the collected data, ensuring synchronization and correlation between the measurements from the PPG sensor and the transmitted data from the wearable devices based on the timestamp.

7. The hub according to claim 5 or 6, wherein at least one of the wearable devices is an ECG sensor, and wherein data collected using the ECG sensor and data collected using the PPG sensor is timestamped and correlated to measure data indicative of the pulse arrival time (PAT).

8. The hub according to claim 7, wherein the ECG sensor is configured to detect electrical activity of the heart and produce an ECG waveform, wherein the PPG sensor is configured to optically measure blood volume changes in the microvascular bed of tissue and produce a PPG waveform, and wherein the internal clock of the hub is configured to provide synchronized timestamping for both the ECG waveform and the PPG waveform, and wherein a processing unit of the hub is configured to calculate the PAT based on the synchronized timestamped data from both the ECG and PPG waveforms.

9. The hub according to claim 8, wherein the processing unit of the hub is configured to derive PAT as the time difference between a point on the ECG waveform, and a point on the PPG waveform.

10. The hub according to any one of the preceding claims, wherein the PPG sensor is configured to measure SpO2 in reflection mode by analyzing light reflected from a fingertip placed over the optical window during use.

11. The hub according to any one of the preceding claims, wherein the hub further comprises a control unit configured to modify measurement schemes for the identified user, wherein said modification includes at least one of adjusting the frequency of measurements, changing the set of parameters to be measured, changing one or more measurement types, and/or altering the duration of measurement periods.

12. The hub according to any one of the preceding claims, wherein the control unit is configured to notify the identified user of drug regimen changes via a communication interface.

13. The hub according to any one of the preceding claims, wherein the hub is usable for recharging wearable devices.

14. A method of configuring a hub wirelessly connectable to a plurality of wearable devices of one or more users, each wearable device being attachable to a part of a body for collecting health-related data, wherein the hub is provided with a photoplethysmogram, PPG, sensor, and wherein an optical window of the PPG sensor is provided on the hub, and wherein a user-identification sensor is provided at the PPG sensor, the user-identification sensor being used to authenticate a user identity.

15. A modular system comprising the hub of any one of the preceding claims 1-13 and modular wearable devices wirelessly connectable to the hub.

16. A method of performing user-authenticated health data collection and communication using a hub and a plurality of wearable devices of one or more users communicatively coupled to the hub, each wearable device being attachable to a part of a body for collecting health-related data, the method comprising the steps of:
establishing a wireless connection between the hub and at least a subset of the plurality of wearable devices of the one or more users;
providing a photoplethysmogram (PPG) sensor integrated within the hub, wherein an optical window of the PPG sensor is arranged on the hub to collect optical data;
activating a user-identification sensor arranged at the PPG sensor; authenticating a user identity based on data received from the user-identification sensor; and
collecting health-related data from the plurality of wearable devices upon successful authentication of the user identity.
